# EUROPÄISCHE PATENTSCHRIFT

(11) **EP 1 161 407 B1**
(45) Veröffentlichungstag und Bekanntmachung des Hinweises auf die Patenterteilung: **04.02.2004**
(21) Anmeldenummer: 00906367.8
(22) Anmeldetag: 22.02.2000
(51) Int. Cl.: C07C 29/42, C07C 29/17

(54) **VERFAHREN ZUR HERSTELLUNG VON ACETYLENALKOHOLEN UND DEREN FOLGEPRODUKTEN**
METHOD FOR PREPARING ACETYLENE ALCOHOLS AND THEIR SECONDARY PRODUCTS
PROCEDE DE PREPARATION D'ALCOOLS D'ACETYLENE ET LEURS PRODUITS SECONDAIRES

(30) Priorität: 22.02.1999 DE 19907532
(43) Veröffentlichungstag der Anmeldung: 12.12.2001
(73) Patentinhaber: BASF AKTIENGESELLSCHAFT, 67056 Ludwigshafen (DE)
(72) Erfinder: BRUNNER, Melanie, D-68165 Mannheim (DE); HENKELMANN, Jochem, D-68165 Mannheim (DE); KAIBEL, Gerd, D-68623 Lampertheim (DE); KINDLER, Alois, Dr., D-67269 Grünstadt (DE); KNOLL, Christian, D-67067 Ludwigshafen (DE); RUST, Harald, D-67435 Neustadt (DE); TRAGUT, Christian, D-67157 Wachenheim (DE)
(74) Vertreter: Isenbruck, Günter, Dr.
(86) Internationale Anmeldenummer: PCT/EP2000/001425
(87) Internationale Veröffentlichungsnummer: WO 2000/050370

(56) Entgegenhaltungen:
- DE-A- 19 500 479
- FR-A- 950 894
- US-A- 2 488 082

## Beschreibung

Die vorliegende Erfindung betrifft ein mehrstufiges Verfahren zur Herstellung von Alkoholen aus Carbonylverbindungen und Alkinen sowie, in einer bevorzugten Ausführungsform, ein integriertes Verfahren zur Herstellung von Alkoholen aus Carbonylverbindungen und Alkinen.

Prinzipiell sind Verfahren zur Herstellung von Alkoholen aus Carbonylverbindungen und Alkinen bekannt. So offenbart z.B. die DE-A 2 008 675 ein Verfahren zur Herstellung von tertiären Acetylenglykolen durch Umsetzung von Acetylen mit Ketonen.

Die FR 950 894 betrifft ein Verfahren zur Herstellung eines Acetylenalkohols, in dem Acetylen mit einem Keton in Gegenwart eines Alkalimetallalkoholats eines primären oder sekundären Alkohols umgesetzt wird, wobei der Alkohol nur ein Sauerstoffatom im Molekül aufweist und bei den gewählten Reaktionstemperaturen in Wasser nicht vollständig löslich ist.

Zhou Hongying beschreibt in Huaxue Shijie 38 (1997) S. 269 - 272 die Herstellung von Methylbutinol durch Umsetzung von Aceton mit Acetylen unter Katalyse durch K-Isobutoxid.

Die GB-A 620,298 betrifft einen cyclischen Prozeß zur Herstellung von Acetylenalkoholen aus Carbonylverbindungen und 1-Alkinen, wobei als Base eine Suspension von feinverteiltem KOH in einem Lösungsmittel verwendet wird. Als Lösungsmittel wird dort ein Acetal oder ein unter Normalbedingungen flüssiger Polyether offenbart.

Eine Aufgabe der vorliegenden Erfindung war es, ein in wirtschaftlicher und ökologischer Hinsicht äußerst effizientes Verfahren zur Herstellung von ungesättigten und gesättigten Alkoholen bereitzustellen.

Demgemäß betrifft die vorliegende Erfindung ein Verfahren zur Herstellung mindestens eines ungesättigten Alkohols (B), das die folgenden Stufen (I) bis (III) umfaßt:
(I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
(II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
(III) Destillation des in Stufe (II) erhaltenen Gemisches (G-II) unter Erhalt des mindestens einen Alkohols (B) sowie eines Gemisches (G-III), umfassend das Lösungsmittel (L) und den Alkohol (A),
wobei das in Stufe (III) erhaltene Lösungmittel (L) und der in Stufe (III) erhaltene Alkohol (A) als Mischung in Stufe (I) rückgeführt werden,
dadurch gekennzeichnet, dass die Stufen (I) bis (III) kontinuierlich durchgeführt werden.

Bezüglich der Herstellung des Gemisches (G-I) in Stufe (I), das das Lösungsmittel (L), den Alkohol (A) und das Alkoholat (AL) umfaßt, existieren keine besonderen Beschränkungen. Es muß lediglich gewährleistet sein, daß (L) und (A) zusammen als Edukte zugegeben werden können.

In einer bevorzugten Ausführungsform wird die Stufe (I) in einer oder mehreren Destillationskolonnen durchgeführt, in der oder in denen das mindestens eine Alkoholat durch azeotrope Trocknung hergestellt wird. Hierbei ist es beispielsweise möglich, (L) und (A) bereits als Gemisch als einen einzigen Eduktstrom zuzuführen. Ebenso ist aber auch möglich, (L) und (A) getrennt zuzuführen und die Ströme erst in der mindestens einen Kolonne zu vereinen. Selbstverständlich ist es auch möglich, mehrere geeignete Lösungsmittel (L) und/oder mehrere geeignete Alkohole (A) einzusetzen. Hierbei ist es denkbar, diese in einem einzigen Eduktstrom der mindestens einen Kolonne zuzuführen oder in zwei oder mehreren Eduktströmen, die die jeweiligen Lösungsmittel (L) und/oder die jeweiligen Alkohole (A) umfassen.

Zusätzlich zu (L) und (A) wird als weiteres Edukt eine wäßrige Lösung eines oder mehrerer Alkali- und/oder Erdalkalihydroxide in einem oder mehreren Eduktströmen zugegeben. Ebenfalls ist in diesem Zusammenhang die Verwendung von Alkali- und/oder Erdalkalihydriden und/oder von Alkaliund/oder Erdalkalialkylverbindungen denkbar.

Sollte es im Rahmen der vorliegenden Erfindung erforderlich sein, können die einzelnen Eduktströme vor der Vermischung selbstverständlich auf eine gewünschte Temperatur gebracht werden. Eine solche Vortemperierung ist nach allen denkbaren Verfahren möglich.

Durch Abdestillieren von Wasser wird im Laufe der Durchführung der Stufe (I) aus dem mindestens einen Alkohol (A) und dem mindestens einen Alkaliund/oder Erdalkalihydroxid eine Mischung gewonnen, die mindestens ein Alkalioder Erdalkali-Alkoholat und das mindestens eine organische Lösungsmittel (L) umfaßt. Diese Mischung wird vorzugsweise aus dem Sumpf der verwendeten Kolonne gewonnen.

Im weiteren Verlauf der Durchführung der Stufe (I) wird aus dieser Mischung durch Destillation der überschüssige, mindestens eine Alkohol (A) abgetrennt. Diese Abtrennung kann sowohl in der gleichen Kolonne durchgeführt werden, in der das mindestens eine Alkoholat hergestellt wurde, als auch in einer oder mehreren nachgeschalteten Kolonnen. In einer bevorzugten Ausführungsform der vorliegenden Erfindung wird Stufe (I) in einer einzigen Kolonne durchgeführt, in der sowohl Wasser als auch der mindestens eine Alkohol (A) abdestilliert werden.

Im Rahmen der vorliegenden Erfindung wird soviel Alkohol (A) abgetrennt, daß das nach der Abtrennung von Wasser und der unvollständigen Abtrennung des mindestens einen Alkohols (A) das resultierende Gemisch im allgemeinen Alkohol (A) im Bereich von 0 bis 55 Gew.-% enthält. Bevorzugt wird jedoch das Verfahren so geführt, daß der Alkohol (A) nicht quantitativ abgetrennt wird. Das Verfahren wird vielmehr so geführt, daß das nach der Abtrennung von Wasser und der unvollständigen Abtrennung des mindestens einen Alkohols (A) das resultierende Gemisch Alkohol (A) im Bereich von 1 bis 55 Gew.-%, bevorzugt im Bereich von 2 bis 10 Gew.-% und besonders bevorzugt ungefähr 5 Gew.-% enthält.

Bei der Abtrennung von Wasser in Stufe (I) ist es denkbar, je nach gewähltem Lösungsmittel (L) und Alkohol (A), daß ein bestimmter Anteil von (L) und/oder
(A) zusammen mit dem Wasser als mehrphasiges Gemisch abdestilliert wird. Ist dies der Fall, so ist es im Rahmen des erfindungsgemäßen Verfahrens möglich, dieses abgetrennte, mehrphasige Gemisch, umfassend Wasser und (L) und/oder (A), einem Phasenscheider zuzuführen und die einzelnen Phasen zu trennen. Damit es möglich, solchermaßen abgetrenntes (L) und/oder (A) als Edukt(e) in Stufe (I) rückzuführen. Auch die solchermaßen abgetrennte wäßrige Phase kann im erfindungsgemäßen Verfahren, wie weiter unten beschrieben, in einer weiteren Stufe des Verfahrens eingesetzt werden.

Als Alkohole (A), die im erfindungsgemäßen Verfahren eingesetzt werden können, sind unter anderem primäre und sekundäre Alkohole mit 4 bis 8 Kohlenstoffatomen wie n-Butanol, Isobutanol, n-Pentanol, 3-Methylbutanol-1, 2-Methylbutanol-1, 2,2-Dimethylpropanol-1, n-Hexanol, 2-Ethylhexanol-1, Butanol-2, Pentanol-2, Pentanol-3, 2-Methylbutanol-3, 2-Methylbutanol-2 oder Cyclohexanol zu nennen. Ebenso sind natürlich auch Verbindungen der allgemeinen Struktur HO-CH₂-CH₂-OR"' denkbar, wobei R"' so gewählt wird, daß die jeweilige Verbindung in dem verwendeten Lösungsmittel(gemisch) löslich ist. Besonders bevorzugt sind hierbei n-Butanol und Isobutanol, insbesondere bevorzugt Isobutanol.

Als organische Lösungsmittel (L) werden im allgemeinen aprotische, bevorzugt polare aprotische Lösungsmittel verwendet. Unter anderem kommen aliphatische, cycloaliphatische und/oder gegebenenfalls substituierte aromatische Kohlenwasserstoffe wie beispielsweise Cyclohexan, Benzol, Toluol, Xylol, Cumol oder p-Diisopropylbenzol, Acetale von Aledhyden und Ketonen, cyclische oder acyclische aliphatische Ether wie beispielsweise symmetrische oder asymmetrische Dialkylether von Ethan oder Butan oder Glykole wie beispielsweise Polyalkylenglykole mit C₂- bis C₆-Alkylresten oder Glykolester zum Einsatz. Ebenso sind beispielsweise Dimethylsulfoxid, N-Methylpyrrolidon, Dimethylformamid oder Hexamethylphosphortriamid geeignet. Besonders bevorzugt sind im Rahmen des erfindungsgemäßen Verfahrens aromatische Kohlenwasserstoffe, und hierbei besonders Xylol.

Als bevorzugte Hydroxide sind im Rahmen des erfindungsgemäßen Verfahrens die Alkalihydroxide zu nennen, wobei KOH besonders bevorzugt ist. Beim Einsatz der wäßrigen Lösung von KOH wird vorzugsweise ein Lösung verwendet, die im allgemeinen 2 bis 60 Gew.-%, bevorzugt 5 bis 50 Gew.-%, besonders bevorzugt 30 bis 35 Gew.-% KOH aufweist.

Das oben beschriebene Gemisch, das noch einen bestimmten Anteil an Alkohol (A) sowie Lösungsmittel (L) und Alkoholat (AL) umfaßt, wird im Anschluß an Stufe (I) der Stufe (II) zugeführt. In dieser Stufe (II) des erfindungsgemäßen Verfahrens wird mindestens eine Carbonylverbindung der allgemeinen Struktur R-CO-R' mit mindestens einem Alkin der allgemeinen Struktur R"-C≡C-H und dem in Stufe (I) erhaltenen Gemisch (G-I) umgesetzt und ein Alkohol (B) erhalten.

In einer bevorzugten Ausführungsform des erfindungsgemäßen Verfahrens wird dazu in einem geeigneten Reakor das aus Stufe (I) erhaltene Gemisch (G-I), umfassend das Lösungsmittel (L), den Alkohol (A) und Alkoholat (AL), vorgelegt und das mindestens eine Alkin und die mindestens eine Carbonylverbindung eingeleitet.

Die Einleitung von Alkin und/oder Carbonylverbindung kann dabei nach allen geeigneten Methoden erfolgen. Möglich ist dabei beispielsweise, Alkin und Carbonylverbindung vor der Einleitung in den Reaktor zu einem Eduktstrom zusammenzuführen und in den Reaktor einzuleiten. Selbstverständlich ist es aber auch möglich, Alkin und Carbonylverbindung getrennt voneinander als einzelne Eduktströme in den Reaktor einzuleiten.

Weiterhin ist es im Falle, daß Alkin und Carbonylverbindung getrennt voneinander als einzelne Eduktströme in den Reaktor eingeleitet werden, möglich, zuerst Alkin einzuleiten und dann Carbonylverbindung. Natürlich ist es auch denkbar, zuerst Alkin einzuleiten und dann, bei anhaltender Einleitung des Alkins, die Carbonylverbindung zuzugeben und Alkin und Carbonylverbindung parallel einzuleiten. In einer bevorzugten Ausführungsform der Erfindung werden Alkin und Carbonylverbindung als getrennte Eduktströme der Stufe (II) gleichzeitig in das Gemisch (G-I) aus Stufe (I) eingeleitet. Das Einleiten kann dabei prinzipiell sowohl diskontinuierlich als auch kontinuierlich erfolgen. Vorzugsweise erfolgt das Einleiten kontinuierlich.

Je nach der gewählten Temperatur, bei der die Umsetzung in Stufe (II) erfolgen soll, kann es sinnvoll sein, die einzelnen Komponenten vor der Zuleitung in den Reaktor bereits auf die erforderliche Temperatur zu bringen, was nach allen Verfahren aus dem Stand der Technik denkbar ist. Insbesondere kann es. erforderlich sein, das aus Stufe (I) erhaltene Gemisch, das aus der Kolonne abgeführt wird, vor der Zuleitung in den Reaktor der Stufe (II) abzukühlen.

Um eine möglichst gute Durchmischung von Alkin, Carbonylverbindung und dem in dem Gemisch aus Stufe (I) enthaltenen, mindestens einen Alkoholat zu erreichen, wird die Reaktionsmischung in Stufe (II) gerührt. Diese Rührung kann prinzipiell nach allen gängigen Verfahren aus dem Stand der Technik erfolgen. Denkbar ist aber auch, durch eine spezielle Art der Einleitung den Durchmischungsvorgang entweder komplett vorzunehmen oder die Rührung damit zu unterstützen.

In einer bevorzugten Ausführungsform des erfindungsgemäßen Verfahrens wird die Reaktionsmischung in Stufe (II) in einer Mischvorrichtung gemischt, wie sie beispielsweise in der DE-C 42 20 239, die diesbezüglich vollumfänglich durch Bezugnahme in den Kontext der vorliegenden Anmeldung einbezogen wird, beschrieben ist. Sämtliche geeignete Ausführungsformen der dort beschriebenen Mischvorrichtung sind denkbar. So können beispielsweise Alkin, Carbonylverbindung und das Gemisch aus Stufe (I) in separaten Strömen in die Mischvorrichtung eingebracht werden, die dementsprechend mindestens 3 Einlaßöffnungen aufweisen muß. Ebenso können (a) Alkin und Carbonylverbindung oder (b) Alkin und Gemisch aus Stufe (I) oder (c) Carbonylverbindung und Gemisch aus Stufe (I) vor dem Einbringen in die Mischvorrichtung gemischt werden, beispielsweise unter Verwendung einer Mischvorrichtung der hier beschriebenen Art, und das resultierende Gemisch als ein Strom und (a) Gemisch aus Stufe (I) oder (b) Carbonylverbindung oder (c) Alkin als weiterer Strom in die Mischvorrichtung eingebracht werden. Dementsprechend muß die Mischvorrichtung in diesem Fall mindestens zwei Einlaßöffnungen aufweisen. Für jede der Ausführungsformen ist es denkbar, daß die einzelnen Ströme, die in die Mischvorrichtung eingebracht werden sollen, vor dem Einbringen durch eine geeignete Vorrichtung, die der Mischvorrichtung vorgeschaltet ist, jeweils in zwei oder mehr Ströme aufgeteilt werden, die anschließend in die Mischvorrichtung eingebracht werden.

Weiter können zur Mischung der einzelnen Komponenten, die in Stufe (II) zur Reaktion gebracht werden sollen, auch zwei oder mehr Mischvorrichtungen der hier beschriebenen Art eingesetzt werden. Beispielsweise können in jeder dieser mindestens zwei Mischvorrichtungen Mischungen hergestellt werden, die anschließend vereinigt werden.

Sollen die Komponenten, die in Stufe (II) zur Reaktion gebracht werden, mit Hilfe dieser Mischvorrichtung lediglich gemischt werden, so ist im Rahmen der vorliegenden Erfindung darauf zu achten, daß die Reaktionsbedingungen in der Mischvorrichtung, wie beispielsweise Temperatur und Druck,derart gewählt werden, daß keine unerwünschten Reaktionen beim Mischvorgang erfolgen.

Bevorzugt wird die Mischvorrichtung als Reaktionsmischpumpe eingesetzt. In der Mischvorrichtung werden die einzelnen Komponenten, die in Stufe (II) zur Reaktion gebracht werden, sowohl gemischt als auch miteinander zur Reaktion gebracht, wie dies untenstehend beschrieben ist.

Generell ist es im Rahmen der vorliegenden Erfindung denkbar, zur Umsetzung von Alkin mit Carbonylverbindung und dem aus der Stufe (I) erhaltenen Gemisch in Stufe (II) zu dem mindestens einen organischen Lösungsmittel (L) und dem mindestens einen Alkohol (A), die in dem aus Stufe (I) erhaltenen Gemisch enthalten sind, zusätzlich ein oder mehrere weitere geeignete Lösungsmittel zuzugeben.

Als Carbonylverbindungen der allgemeinen Struktur R-CO-R' werden im erfindungsgemäßen Verfahren beispielsweise aliphatische, araliphatische oder heterocyclische Ketone mit bis zu 30 C-Atomen eingesetzt. Dabei könne R und R' gleich oder verschieden sein, und entweder zwei getrennte Reste oder aber verbrückt sein. Ebenso ist es möglich, daß die Reste R und/oder R' olefinische oder acetylenische Funktionen aufweist. Beispielhaft seien Aceton, Isobutylmethylketon, 6,10-Dimethyl-5-undecen-2-on, 6,11,14-Trimethyl-2-Pentadecanon, 2-Methyl-2-hepten-6-on, 2-Methylheptan-6-on, [4(2,6,6-Trimethyl-1-cyclohexenyl)-3-buten-2-on], Methylethylketon, Cyclohexanon, Acetophenon, Benzophenon, Piperidon-4 genannt, wobei Aceton, Isobutylmethyketon, 6,10-Dimethyl-5-undecen-2-on, 6,11,14-Trimethyl-2-Pentadecanon, 2-Methyl-2-hepten-6-on, 2-Methylheptan-6-on und [4(2,6,6-Trimethyl-1-cyclohexenyl)-3-buten-2-on] bevorzugt sind.

Auch der Einsatz von Aldehyden, die keinen aciden Wasserstoff aufweisen, ist prinzipiell denkbar. Hierbei sind unter anderem Aldehyde RCHO zu nennen, wobei R = H oder ein Alkylrest mit bis zu 30 C-Atomen ist. Beispielhaft seien etwa Ethylhexanal oder CH₂O genannt. Als bevorzugt verwendeter Aldehyd ist CH₂O zu nennen.

Als Alkine der allgemeinen Struktur R"-C≡C-H seien solche genannt, bei denen R" ausgewählt wird aus der Gruppe bestehend aus Wasserstoff und einem aliphatischen, araliphatischen oder aromatischen Rest mit bis zu 15 C-Atomen. Als Beispiele seien etwa Acetylen, Propin, 1-Butin, 1-Pentin, 1-Hexin, Phenylacetylen, Benzylacetylen, 3-Methyl-1-butin oder Verbindungen wie etwa 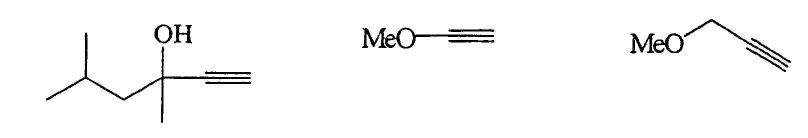 genannt.

Wird im erfindungsgemäßen Verfahren Acetylen als Alkin eingesetzt, so ist es denkbar, durch Umsetzung mit entsprechenden Mengen an Carbonylverbindung und Alkoholat sowohl Alkin-Monoole als auch Alkin-Diole herzustellen. Bei Einsatz eines Alkins, bei dem R" ≠ H, werden Alkin-Monoole erzeugt.

Selbstverständlich ist es im Rahmen des erfindungsgemäßen Verfahrens auch denkbar, als Alkin der allgemeinen Struktur R"-C≡C-H ein Alkin-Monool einzusetzen und durch Umsetzung mit der Carbonylverbindung und dem Alkoholat daraus ein Alkin-Diol herzustellen. Beispielsweise sei hier etwa die Umsetzung von Aceton mit Methylbutinol genannt.

Bevorzugt wird als Alkin der allgemeinen Struktur R"-C≡C-H im erfindungsgemäßen Verfahren Acetylen verwendet. Aus den oben als bevorzugt genannten verwendeten Carbonylverbindungen sind damit u.a. die folgenden Alkinole Verbindungen herstellbar, wobei der Begriff "Alkinol" prinzipiell alle Verbindungen umfaßt, die sowohl mindestens eine C-C-Dreifachbindung als auch eine oder mehrere Hydroxylgruppen aufweisen:

2,5-Dimethylhex-3-in-2,5-diol (DMHDY), 3-Methyl-3-hydroxy-but-1-in, 2,4,7,9-Tetramethyl-4,7-dihydroxy-dec-5-in, 3,7,11-Trimethyl-6-dodecan-1-in-3-ol, 3,7,11,15-Tetramethyl-1-hexadecin-3-ol, 3,7-Dimethyl-oct-1-in-6-en-3-ol, 3,7-Dimethyl-oct-1-in-3-ol, 1-Penten-4-in-3-ol, Propinol, But-2-in1-ol, But-1-in-3-ol, oder die Verbindungen

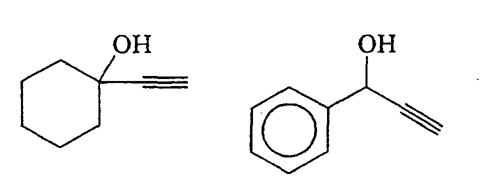

Bei der Herstellung des ungesättigten Alkohols kann das stöchiometrische Verhältnis von Carbonylverbindung zu Alkin im wesentlichen beliebig gewählt werden.

Werden beispielsweise Alkindiole aus Carbonylverbindung und Acetylen hergestellt, wird bevorzugt ein stöchiometrisches Verhältnis Carbonylverbindung : Acetylen im Bereich von 1,9: 1 bis 2,1 : 1, besonders bevorzugt von etwa 2 : 1 gewählt. Das stöchiometrische Verhältnis Alkoholat : Carbonylverbindung liegt bevorzugt im Bereich von 0,9 : 1 bis 2,1 : 1, besonders bevorzugt im Bereich von 1 : 1 bis 1,5 : 1 und insbesondere bei etwa 1;1 : 1.

Werden beispielsweise Alkinmonoole aus Carbonylverbindung und Acetylen hergestellt, wird bevorzugt ein stöchiometrisches Verhältnis Carbonylverbindung : Acetylen im Bereich von 1 : 1 bis 0,5 : 1, besonders bevorzugt von etwa 0,6 : 1 gewählt. Das stöchiometrische Verhältnis Alkoholat : Carbonylverbindung liegt bevorzugt im Bereich von 1 : 1 bis 0,2 : 1 und insbesondere bei etwa 0,3 : 1.

Die Reaktionstemperatur bei der Umsetzung entsprechender Edukte zu Alkindiolen liegt im Rahmen des erfindungsgemäßen Verfahrens bevorzugt im Bereich von 0 bis 50 °C, weiter bevorzugt im Bereich von 10 bis 40 °C und insbesondere bevorzugt im Bereich von 25 bis 35 °C.

Die Reaktionstemperatur bei der Umsetzung entsprechender Edukte zu Alkinmonoolen liegt im Rahmen des erfindungsgemäßen Verfahrens bevorzugt im Bereich von 0 bis 50 °C, weiter bevorzugt im Bereich von 0 bis 35 °C und insbesondere bevorzugt im Bereich von 0 bis 20 °C.

Die Drücke bei den genannten Umsetzungen liegen bei der Herstellung von Alkinmonoolen und Alkindiolen im Rahmen des erfindungsgemäßen Verfahrens bevorzugt im Bereich von 1 bis 20 bar, weiter bevorzugt im Bereich von 1 bis 5 bar und insbesondere bei 1 bar.

Die Ausbeute an ungesättigtem Alkohol (B), die im Rahmen des erfindungsgemäßen Verfahrens in Stufe (II) erhalten wird, liegt bevorzugt bei mindestens 75 %, weiter bevorzugt bei mindestens 80 %, besonders bevorzugt bei mindestens 85 % und insbesondere bevorzugt bei mindestens 90 %.

Wie bereits oben beschrieben, wird die Umsetzung gemäß Stufe (II) vorzugsweise in einer Reaktionsmischpumpe durchgeführt, wie sie in der DE-C 42 20 239 beschrieben ist, wobei die Ausführungsform der Reaktionsmischpumpe nicht auf die dort offenbarte Mischvorrichtung beschränkt ist. Sämtliche denkbaren Ausführungsformen einer Vorrichtung, in der die Edukte der Stufe (II) sowohl gemischt als auch zur Reaktion gebracht werden können, sind ebenso denkbar.

Letztendlich erhält man in dieser Stufe (II) ein Gemisch (G-II), das mindestens einen ungesättigten Alkohol (B) sowie (A) und (L) umfaßt.

In einer weiter bevorzugten Ausführungform der vorliegenden Erfindung schließt sich an Stufe (II), aus der mindestens ein ungesättigter Alkohol (B) erhalten wird, eine Stufe (II') an, in der der mindestens eine ungesättigte Alkohol hydriert wird.

Demgemäß betrifft die vorliegende Erfindung auch ein Verfahren zur Herstellung mindestens eines hydrierten Alkohols (C), das die folgenden Stufen (I) bis (III') umfaßt :
(I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
(II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
(II') Hydrierung mindestens eines ungesättigten Alkohols (B) in dem aus Stufe (II) erhaltenen Gemisch (G-II) unter Erhalt eines Gemisches (G-II'), umfassend mindestens einen hydrierten Alkohol (C), den Alkohol (A) und das Lösungsmittel (L);
(III') Destillation des in Stufe (II') erhaltenen Gemisches (G-II') unter Erhalt des mindestens einen Alkohols (C), sowie eines Gemisches (G-III'), umfassend das Lösungsmittel (L) und den Alkohol (A),
dadurch gekennzeichnet, daß das in Stufe (III') erhaltene Lösungsmittel (L) und der in Stufe (III') erhaltene Alkohol (A) als Mischung in Stufe (I) rückgeführt werden.

Da in Stufe (II) ein Alkin-Monool und/oder ein Alkin-Diol, je nach Wahl der Reaktionspartner, gebildet wird, sind im Rahmen des vorliegenden Verfahrens verschiedene Hydrierungen denkbar. Wurde beispielsweise ein Alkin-Monool oder ein Alkin-Diol erhalten, so kann das Alkin-Monool oder Alkin-Diol in einer partiellen Hydrierung zum entsprechenden Alkenol hydriert werden. Es ist aber auch möglich, durch entsprechende Wahl der Hydrierbedingungen das jeweilige Alkanol herzustellen.

Die jeweilige Hydrierung kann hierbei prinzipiell nach allen geeigneten Verfahren gemäß dem Stand der Technik durchgeführt werden. So kann die jeweilige Hydrierung in einem Reaktor oder aber in mehreren hintereinander geschalteten Reaktoren durchgeführt werden. Jeder Reaktor kann dabei in allen denkbaren Fahrweisen betrieben werden, wobei vor allem Riesel- und Sumpffahrweise im Festbettreaktor genannt sein sollen. In einer bevorzugten Ausführungsform findet die Hydrierung in zwei hintereinander geschalteten Rohrreaktoren (Festbett) statt, von denen der erste rückvermischt in Rieselfahrweise, der zweite im geraden Durchgang in Riesel- oder Sumpffahrweise betrieben wird.

Hierbei ist es möglich, daß die Komponenten, die in den einen Hydrierreaktor oder in die mehreren Hydrierreaktoren eingeleitet werden, vor der Einleitung vorgeheizt oder auch vorgekühlt werden. Dies kann beispielsweise in einem oder mehreren Wärmetauschern erfolgen.

Weiter ist es denkbar, die Temperatur des Hydrierreaktors oder der Hydrierreaktoren selbst zu regeln, was nach allen Verfahren nach dem Stand der Technik durchgeführt werden kann. Dadurch ist es beispielsweise möglich, eine nachlassende Hydrieraktivität des mindestens einen, zur Hydrierung eingesetzten Katalysators, wie unten beschrieben, durch Erhöhung der Reaktortemperatur zu kompensieren. Diese Erhöhung der Reaktortemperatur kann beispielsweise dadurch erfolgen, daß bei einer exothermen Hydrierungsreaktion die Kühlung des Reaktors vermindert wird. Ebenso ist es natürlich denkbar, die Reaktortemperatur aktiv durch Erwärmung von außen zu erhöhen.

Wird im erfindungsgemäßen Verfahren ein Alkinol zu einem Alkenol hydriert, so können hierfür sämtliche geeigneten, aus dem Stand der Technik bekannten Katalysatoren eingesetzt werden. Als mögliche Katalysatoren sind hierbei unter anderem etwa Pd oder Pd/Pb auf CaCO₃ (Lindlar-Katalysator) zu nennen. Gegebenenfalls werden die Katalysatoren wie beispielsweise Pd zur Erzielung guter Selektivitäten z.B. durch CO partiell vergiftet.

Wird im erfindungsgemäßen Verfahren ein Alkinol zu einem Alkanol hydriert, so können hierfür ebenfalls sämtliche geeignete, aus dem Stand der Technik bekannten Katalysatoren verwendet werden. Bekannte Katalysatoren sind beispielsweise Pd-, Pt-, Ni- (auch Raney-Nickel), Co-, Ru- oder Rh-Katalysatoren, wobei diese geträgert oder als Vollkontaktkatalysatoren eingesetzt werden können. Als Träger können hierbei alle geeigneten gängigen Träger wie beispielsweise Al₂O₃, SiO₂ oder C verwendet werden.

In einer bevorzugten Ausführungsform des Verfahrens, in der ein oder mehrere Alkinole zu den entsprechenden Alkanolen hydriert werden, werden als Katalysatoren geträgerte Katalysatoren oder Vollkontakte verwendet. Als aktives Hydriermetall sind hierbei vor allem die Metalle der 1. 7. und 8. Nebengruppe des Periodensystems zu nennen. Vorzugsweise werden dabei Ni, Ru, Pd, Pt und Rh eingesetzt.

In einer besonders bevorzugten Ausführungsform bei der Hydrierung von Alkinolen zu Alkanolen wird im Rahmen des erfindungsgemäßen Verfahrens als Katalysator ein geträgerter Pd-Katalysator verwendet, wobei das Trägermaterial Aluminiumoxid umfaßt.

Die Hydrierung der Alkinole zu den jeweiligen Alkanolen findet im erfindungsgemäßen Verfahren bei Drücken von im allgemeinen 1 bis 300 bar, bevorzugt 10 bis 200 bar, besonders bevorzugt 15 bis 100 bar und insbesondere bevorzugt 20 bis 50 bar statt.

Im allgemeinen liegen die Temperaturen bei der Hydrierung im erfindungsgemäßen Verfahren bei der Hydrierung von Alkinolen zu den jeweiligen Alkanolen im allgemeinen im Bereich von 30 bis 250 °C, bevorzugt im Bereich von 50 bis 200 °C und besonders bevorzugt im Bereich von 80 bis 160 °C.

Werden im erfindungsgemäßen Verfahren Alkinole zu den jeweiligen Alkenolen hydriert, so liegen die Temperaturen bei der Hydrierung im allgemeinen im Bereich von 30 bis 200 °C, bevorzugt im Bereich von 30 bis 150 °C und besonders bevorzugt im Bereich von 50 bis 130 °C.

Werden im erfindungsgemäßen Verfahren zwei oder mehr hintereinander geschaltete Reaktoren bei der Hydrierung eingesetzt, so ist es denkbar, daß in den einzelnen Reaktoren verschiedene Drucke und/oder verschiedene Temperaturen zur Hydrierung eingestellt werden. So ist es unter anderem möglich, die Temperatur eines Produktstroms aus einem ersten Reaktor, der aufgrund freiwerdender Reaktionswärme eine höhere Temperatur aufweist als der Eduktstrom in diesen ersten Reaktor, vor dem Einleiten in einen zweiten Reaktor nicht zu regeln. Natürlich ist es aber auch möglich, beispielsweise über eine zwischen diesen beiden Reaktoren vorgesehene Zwischenkühlung den Produktstrom aus dem ersten Reaktor vor dem Einleiten in den zweiten Reaktor abzukühlen. So ist es beispielsweise möglich, die Hydriertemperatur im zweiten Reaktor individuell an die Aktivität des dort verwendeten Katalysators anzupassen.

Selbstverständlich ist es auch denkbar, aus dem Alkinol eine Mischung aus Alkenol und Alkanol herzustellen. Dies ist beispielsweise durch entsprechende Wahl der Hydrierbedingungen möglich. Eine weitere Möglichkeit hierfür besteht darin, den Produktstrom aus Stufe (II), der aus dem in Stufe (II) erhaltenen Gemisch besteht, in zwei oder mehr Ströme zu teilen und jeden Strom in separaten Reaktoren zu hydrieren, wobei dann die Hydrierbedingungen in jedem Reaktor unterschiedlich sein können und somit auf einfache Art und Weise Alkenole und Alkanole aus dem Alkinol hergestellt werden können.

Je nach Hydrierbedingungen können aus den bereits oben beschriebenen, bevorzugt hergestellten Alkinolen folgende Alkenole und/oder Alkanole hergestellt werden:

2,5-Dimethylhexan-2,5-diol (DMHD), 3-Methyl-3-hydroxy-but-1-en, 2-Methyl-2-hydroxy-butan, 3,7,11-Trimethyl-3-hydroxy-1,6-dodecadien, 3,7,11,15-Tetramethyl-hexadec-1-en-3-ol, 3,7-Dimethyl-1,6-octadien-3-ol, 3,7-Dimethyl-oct-1-en-3-ol, 3-Methyl-1-(2,6,6-Trimethyl-1-cyclohexen-1-yl), 1,4-Pentadien-3-ol, 3-Methyl-1-(2,6,6-trimethyl-1-cyclohexen-1-yl).

Natürlich ist die Stufe (II') nicht auf eine Hydrierung der Dreifachbindung des aus der Stufe (II) erhaltenen mindestens einen Alkinols beschränkt. Je nach der chemischen Struktur des in Stufe (II) eingesetzten mindestens einen Alkins und/oder der mindestens einen Carbonylverbindung ist es denkbar, daß in Stufe (II') die Reste R und/oder R' und/oder R" chemisch modifiziert werden, indem z.B. Reaktionen an in diesen Resten enthaltenen funktionellen Gruppen durchgeführt werden. Weiter ist es im Rahmen des erfindungsgemäßen Verfahrens auch denkbar, daß solche Umsetzungen zusätzlich zu den weiter oben beschriebenen Hydrierungen durchgeführt werden.

In einer weiter bevorzugten Ausführungsform des erfindungsgemäßen Verfahrens wird die Stufe (II), wie oben beschrieben, in Einzelschritten (i) bis (vi) durchgeführt. Demgemäß betrifft die vorliegende Erfindung auch ein Verfahren, wie oben beschrieben, dadurch gekennzeichnet, daß die Stufe (II) die folgenden Schritte (i) bis (vi) umfaßt:
(i) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt einer Mischung (M-i);
(ii) Hydrolyse der aus Schritt (i) erhaltenen Mischung (M-i) unter Erhalt einer mehrphasigen Mischung (M-ii), umfassend mindestens eine organische Phase;
(iii) Abtrennung der mindestens einen organischen Phase aus der in Schritt (ii) erhaltenen mehrphasigen Mischung (M-ii);
(iv) Extraktion der mindestens einen in Schritt (iii) abgetrennten organischen Phase;
(v) Neutralisation der mindestens einen, in Schritt (iv) extrahierten organischen Phase unter Erhalt einer Mischung (M-v), umfassend mindestens ein Alkalioder Erdalkalisalz;
(vi) Abtrennung des mindestens einen Alkali- oder Erdalkalisalzes aus der in Schritt (v) erhaltenen Mischung (M-v) unter Erhalt eines Gemisches (G-II), umfassend mindestens den Alkohol (A), das Lösungsmittel (L) und einen ungesättigten Alkohol (B).

Aus der Umsetzung der mindestens einen Carbonlyverbindung mit dem mindestens einen Alkin und dem in Stufe (I) erhaltenen Gemisch (G-I), wie oben beschrieben, resultiert zunächst eine Mischung (M-i), die dem Schritt (ii) zugeführt wird. In diesem Schritt (ii) wird der mindestens eine ungesättigte Alkohol (B) durch Hydrolyse freigesetzt.

Für die Art und Weise der Durchführung der Hydrolyse bestehen prinzipiell keine Beschränkungen. So kann diese beispielsweise in einem oder mehreren Reaktoren durchgeführt werden, wobei die Temperatur im jeweiligen Reaktor gegebenenfalls geregelt werden kann. Denkbar ist beispielsweise eine Kühlung durch Sole. Ebenso denkbar ist auch eine Kühlung des Hydrolysewassers, die ebenfalls nach allen denkbaren Verfahren gemäß dem Stand der Technik durchgeführt werden kann. Im allgemeinen wird bei der Durchführung der Hydrolyse die Reaktionsmischung gerührt, wobei diese nach allen gängigen Verfahren nach dem Stand der Technik erfolgen kann. Beispielsweise kann zum Rühren und Mischen eine Mischvorrichtung, wie sie obenstehend beschrieben ist und beispielsweise in der DE-C 42 20 239 offenbart ist, eingesetzt werden.

Die wäßrige Phase, die zu diesem hydrolytischen Schritt (ii) eingesetzt wird, resultiert in einer besonders bevorzugten Ausführungsform des erfindungsgemäßen Verfahrens aus der Extraktion in Schritt (iv). Selbstverständlich kann daneben noch weiteres Wasser zugesetzt werden. Sowohl das zur Hydrolyse verwendete Wasser als auch die zu hydrolisierende Reaktionsmischung können vor der Einleitung in den mindestens einen Hydrolysereaktor auf die gewünschte Temperatur gebracht werden, was beispielsweise durch Wärmetauscher erfolgen kann.

Aus der Hydrolyse in Schritt (ii) wird eine Mischung (M-ii) erhalten, die aus mindestens zwei Phasen besteht, aus mindestens einer organischer Phase und mindestens einer wäßrigen Phase. Aus dieser Mischung (M-ii) wird in Schritt (iii) die mindestens eine organische Phase abgetrennt. Diese Phasentrennung kann prinzipiell nach allen gängigen Methoden erfolgen. Dabei ist es denkbar, daß bei Vorliegen von zwei oder mehr organischen Phasen diese zusammen als mehrphasige organische Mischung abgetrennt werden oder als zwei oder mehr separate organische Phasen. Ebenfalls denkbar ist es, bei Vorliegen von zwei oder mehr wäßrigen Phasen diese zusammen als mehrphasige wäßrige Mischung abzutrennen oder als zwei oder mehr separate wäßrige Phasen.

In einer bevorzugten Ausführungsform der vorliegenden Erfindung wird die hydrolysierte Mischung (M-ii) aus Schritt (ii) in ein oder mehrere Phasentrenngefäße geleitet, die so ausgelegt sein können, daß die Temperatur der zu aufzutrennenden mehrphasigen Mischung im Trenngefäß regelbar ist. Vorzugsweise findet die Phasentrennung bei Temperaturen statt, die im allgemeinen im Bereich von 10 bis 80 °C, bevorzugt im Bereich von 20 bis 60 °C und besonders bevorzugt im Bereich von 40 °C liegen.

In einer besonders bevorzugten Ausführungsform des erfindungsgemäßen Verfahrens wird die mindestens eine wäßrige Phase, die einen Gehalt an Alkaliund/oder Erdalkalihydroxid im Bereich von 2 bis 60 Gew.-%, bevorzugt im Bereich von 30 bis 35 Gew.-% aufweist, als Edukt in Stufe (I) rückgeführt. Um bei der in Stufe (I) erfolgenden Alkoholatherstellung eine Basenkonzentration zu erreichen, die die Herstellung des mindestens eines Alkoholates (AL) ermöglicht, kann es notwendig sein, daß in Stufe (I), zusätzlich zu der aus Schritt (iii) erhaltenen wäßrigen, Alkali- und/oder Erdalkalihydroxid umfassenden mindestens einen wäßrigen Phase, eine weitere wäßrige Lösung, die Alkali- und/oder Erdalkalihydoxid umfaßt, zugesetzt wird.

Die mindestens eine organische Phase wird in einem nächsten Schritt (iv) extrahiert. Wurde in Schritt (iii) eine einzige organische Phase oder eine mehrere organische Phasen umfassende Mischung abgetrennt, so wird diese Phase oder diese Mischung extrahiert. Wurden mehrere organische Phasen abgetrennt, so ist es beispielsweise denkbar, jede einzelne organische Phase separat zu extrahieren.

In einer bevorzugten Ausführungsform der vorliegenden Erfindung wird zur Extraktion die wäßrige Phase eingesetzt, die aus der Stufe (I) durch Destillation erhalten wird. Dabei ist es natürlich denkbar, daß dieser wäßrigen Phase zusätzliches Wasser zugesetzt wird.

Die Extraktion kann nach allen möglichen Verfahren, die aus dem Stand der Technik bekannt sind, beispielsweise unter Verwendung einer Mischvorrichtung, wie sie obenstehend beschrieben und in der DE-C 42 20 239 offenbart ist, durchgeführt werden. Hierbei ist unter anderem die Verwendung von Kolonnen wie Siebboden-Kolonnen, pulsierende Hebboden-Kolonnen oder Füllkörperkolonnen zu nennen. In einer bevorzugten Ausführungsform wird die Extraktion im Gegenstromverfahren durchgeführt, wobei die Temperatur des Extraktionsgutes bei der Extraktion im allgemeinen im Bereich von 30 bis 50 °C, bevorzugt bei ungefähr 40 °C liegt.

Demgemäß betrifft die vorliegende Erfindung auch ein Verfahren, wie oben beschrieben, dadurch gekennzeichnet, daß die Extraktion in Schritt (iv) als Gegenstromextraktion durchgeführt wird.

In diesem Extraktionsschritt (iv) wird der mindestens einen organischen Phase das mindestens eine Alkali- oder Erdalkalihydroxid soweit entzogen, daß der Gehalt der mindestens einen organischen Phase an Alkali- und/oder Erdalkalihydroxid weniger als 1 Gew.-%, bevorzugt weniger als 0,1 Gew.-% und besonders bevorzugt weniger als 0,01 Gew.-% beträgt.

Die wäßrige Phase, die aus der Extraktion resultiert, wird in einer bevorzugten Ausführungsform des vorliegenden erfindungsgemäßen Verfahrens in Schritt (ii) rückgeführt.

Die mindestens eine extrahierte organische Phase kann in dem weiteren Schritt (v) neutralisiert werden. Die Neutralisation erfolgt hierbei durch Zusatz von Säure, wobei als Säuren u.a. Phosphorsäure, Ameisensäure, Essigsäure, Schwefelsäure oder Kohlensäure zu nennen sind. Ebenso denkbar ist die Verwendung von festem Kohlendioxid. Bevorzugt wird im erfindungsgemäßen Verfahren Phosphorsäure eingesetzt.

In dem folgenden Schritt (vi) wird das bei der Neutralisation entstandene, mindestens eine Alkali- oder Erdalkalisalz aus der aus Schritt (v) erhaltenen Mischung (M-v) abgetrennt. Diese Abtrennung kann generell nach allen aus dem Stand der Technik bekannten Verfahren erfolgen. Die Salzabtrennung kann im erfindungsgemäßen Verfahren unterbleiben, wenn das Produkt, von dem Salz abgetrennt wird, thermisch relativ wenig belastet wird. Dies ist beispielsweise dann der Fall, wenn der mindestens eine ungesättigte Alkohol (B) nicht hydriert wird.

In einer bevorzugten Ausführungsform des erfindungsgemäßen Verfahrens erfolgt die Abtrennung des mindestens einen anorganischen Salzes aus der mindestens einen in Schritt (v) erhaltenen Mischung (M-v) über Totalverdampfung der organischen Substanzen durch Verwendung beispielsweise eines Dünnschichtverdampfers oder eines Fallfilmverdampfers. Das mindestens eine zurückbleibende anorganische Salz wird mittels einer oder mehrerer geeigneter Substanzen aus dem Verdampfer ausgespült. Als hierfür geeignete Substanzen seien unter anderem Polyalkylenglykole wie etwa Polyethylenglykol genannt. In einer besonders bevorzugten Ausführungsform wird von den Substanzen, die zum Ausspülen des mindestens einen anorganischen Salzes eingesetzt wird, ein Anteil von mindestens 0,5 %, bevorzugt 1 bis 2 % und besonders bevorzugt mindestens 1 bis 10% rückgeführt.

Sollte der mindestens eine ungesättigte Alkohol (B) im erfindungsgemäßen Verfahren nicht hydriert werden oder ist der mindestens eine hydrierte Alkohol (C) nicht ausreichend thermostabil, so daß bei den bei der Totalverdampfung auftretenden Temperaturen ein Verlust an Wertprodukt auftritt, so kann die Salzabtrennung auch über Ionentausch erfolgen. Bezüglich des Ionentausches sind alle geeigneten Verfahren, die aus dem Stand der Technik bekannt sind, denkbar.

In einer weiter bevorzugten Ausführungsform wird zusätzlich zu der aus Schritt (v) erhaltenen Mischung (M-v) derjenige Hochsiedersumpf in den Verdampfer geleitet, der bei der Destillation in Stufe (III) anfällt. Damit wird erreicht, daß Reste an Wertprodukt, die in dem Hochsiedersumpf enthalten sind, in den Prozeß zurückgeführt werden. Ein weiterer Vorteil, der mit dieser Rückführung verbunden ist, ist die Tatsache, daß der genannte Hochsiedersumpf als Schmiermittel für den Verdampfer wirkt.

Das Verdampferdestillat, das aus der Totalverdampfung der organischen Substanzen resultiert, wird im erfindungsgemäßen Verfahren kondensiert. In einer bevorzugten Ausführungsform des erfindungsgemäßen Verfahrens erfolgt diese Kondensation in mindestens zwei, in einer weiter bevorzugten Ausführungsform in zwei Schritten. Die Temperatur, bei der der erste Kondensationsschritt erfolgt, liegt im allgemeinen im Bereich von 30 bis 80 °C, bevorzugt im Bereich von 35 bis 50 °C und weiter bevorzugt bei 40 °C. Die Temperatur, bei der der zweite Kondensationsschritt erfolgt, liegt im allgemeinen im Bereich von 0 bis 40 °C, bevorzugt im Bereich von 5 bis 10 °C und weiter bevorzugt bei etwa 10 °C.

Das Gemisch (G-II), das nach den Kondensationsschritten anfällt und das den mindestens einen ungesättigten Alkohol (B), (L) und (A) umfaßt, wird dann der Stufe (II') oder (III) zugeführt. Dabei ist es denkbar, daß, im Falle mehrerer Kondensationsschritte, nur das Kondensat eines Kondensationsschrittes in Stufe (II') und/oder (III) weiterverarbeitet wird. Es ist aber auch möglich, daß mehrere Kondensatströme zusammen in Stufe (II') und/oder (III) weiterverarbeitet werden.

In Stufe (III) der vorliegenden Erfindung wird das aus der vorhergehenden Stufe erhaltene Gemisch (G-II') oder (G-II') destilliert und bei dieser Destillation der mindestens eine, in der vorhergehenden Stufe hergestellte Alkohol (B) oder (C) erhalten. Ebenso werden auch das mindestens eine organische Lösungsmittel (L) und der mindestens eine Alkohol (A) abgetrennt. Diese Destillation kann wiederum nach allen gängigen Verfahren, die aus dem Stand der Technik bekannt sind, erfolgen. In einer bevorzugten Ausführungsform des erfindungsgemäßen Verfahrens wird die Destillation in einer Packungskolonne mit Trennwand durchgeführt.

Die nach der Destillation in Stufe (III) erhaltenen (L) und (A) werden nach dem erfindungsgemäßen Verfahren als Edukte in die Stufe (I) rückgeführt, wobei (L) und (A) getrennt voneinander oder als Mischung in Stufe (I) rückgeführt werden können.

In einer weiteren bevorzugten Ausführungsform des vorliegenden Verfahrens wird der aus Stufe (III) bzw. (III') durch Destillation erhaltene, in der Stufe (II) bzw. in den Stufen (II) und (II') hergestellte, mindestens eine Alkohol in einer weiteren Stufe (IV) konfektioniert.

Demgemäß betrifft die vorliegende Erfindung auch ein Verfahren, wie oben beschrieben, das die weitere Stufe (IV) umfaßt:
(IV) Konfektionierung des mindestens einen Alkohols B oder C, der in der Stufe (II) oder in den Stufen (II) und (II') hergestellt und in Stufe (III) oder in Stufe (III') erhalten wird.

Die Konfektionierung kann nach allen gängigen und bekannten Methoden gemäß dem Stand der Technik erfolgen und kann prinzipiell darauf abgestellt werden, welche Methode zur Verarbeitung am günstigsten ist und in welcher Form das konfektionierte Material vorliegen soll.

Jede der Stufen (I), (II), (II'), (III), (III') und (IV) sowie jeder der Schritte (i) bis (vi) kann prinzipiell kontinuierlich oder diskontinuierlich durchgeführt werden. In einer bevorzugten Ausführungsform des erfindungsgemäßen Verfahrens sind diese Stufen und Schritte so konzipiert, daß jede Stufe und jeder Schritt kontinuierlich durchgeführt werden kann.

Demgemäß betrifft die vorliegende Erfindung auch ein Verfahren, wie oben beschrieben, dadurch gekennzeichnet, daß jede Stufe und jeder Schritt als kontinuierlicher Prozeß erfolgt.

Ebenso betrifft die vorliegende ein Verfahren wie oben beschrieben, dadurch gekennzeichnet, daß die Reste R und R' der Carbonylverbindung der allgemeinen Struktur R-CO-R' gleich oder unterschiedlich sind und geradkettige, verzweigtkettigte oder cyclische, gegebenfalls ungesättigte aliphatische Reste sind.

Als bevorzugte Carbonylverbindung nach diesem Verfahren werden in der vorliegenden Erfindung Aceton und Methyl-iso-Butylketon eingesetzt. Daher betrifft die vorliegende Erfindung auch ein Verfahren, wie oben beschrieben, dadurch gekennzeichnet, daß die Carbonylverbindung der allgemeinen Struktur R-CO-R' Aceton oder Methyl-iso-Butylketon ist.

Als bevorzugt verwendetes Alkin der allgemeinen Struktur wird im erfindungsgemäßen Verfahren Acetylen eingesetzt. Demgemäß betrifft die vorliegende Erfindung auch ein Verfahren, wie oben beschrieben, dadurtch gekennzeichnet, daß als als Alkin der allgemeinen Struktur R"-C≡C-H Acetylen eingesetzt wird.

Wie obenstehend ausführlich beschrieben, stellt die vorliegende Errfindung ein Verfahren zur Herstellung mindestens eines Alkohols zur Verfügung, in dem eine große Anzahl von Produkten, die aus den einzelnen Stufen und Schritten resultieren, wieder in das Verfahren rückgeführt werden. Damit wird ein kostensparendes und ökologisch effizientes Verfahren bereitgestellt, was durch die bevorzugte kontinuierliche Betriebsweise jeder Stufe und jedes Schrittes noch verbessert wird.

Die vorliegende Erfindung betrifft daher auch ein integriertes Verfahren zur Herstellung mindestens eines ungesättigten Alkohols (B), das die folgenden kontinuierlich durchgeführten Stufen (a) bis (h) umfaßt:
(a) Umsetzung mindestens eines Alkali- oder Erdalkalihydroxids mit mindestens einem Alkohol (A) in mindestens einem organischen Lösungsmittel (L) unter Verwendung der in Stufe (d) erhaltenen wäßrigen Phase (P-d) und unter Erhalt eines Gemisches (G-a), umfassend mindestens das Lösungsmittel (L), den Alkohol (A) und ein Alkoholat (AL), und unter Erhalt einer wäßrigen Phase (P-a), die der Stufe (e) zugeführt wird;
(b) Umsetzung mindestens einer Carbonylverbindung der allgemeinen Struktur R-CO-R' mit mindestens einem Alkin der allgemeinen Struktur R"-C≡C-H und dem in Stufe (a) erhaltenen Gemisch (G-a) unter Erhalt eines Gemisches (G-b), umfassend mindestens einen ungesättigten Alkohol (B), wobei die Umsetzung vorzugsweise in einer Reaktionsmischpumpe durchgerührt wird;
(c) Hydrolyse des Gemisches (G-b) aus Stufe (b) unter Verwendung der in Stufe (e) erhaltenen wäßrigen Phase (P-e) unter Erhalt einer mehrphasigen Mischung (M-c), umfassend mindestens eine organische Phase und mindestens eine wäßrige Phase;
(d) Abtrennung der mindestens einen organischen Phase aus der in Stufe (c) erhaltenen mehrphasigen Mischung (M-c) unter Erhalt mindestens einer wäßrigen Phase (P-d), die in Stufe (a) rückgeführt wird;
(e) Gegenstromextraktion der in Stufe (d) abgetrennten mindestens einen organischen Phase unter Verwendung der in Stufe (a) erhaltenen wäßrigen Phase (P-a) und unter Erhalt einer wäßrigen Phase (P-e), die in Stufe (c) rückgeführt wird;
(f) Neutralisation der mindestens einen, in Stufe (e) erhaltenen organischen Phase unter Erhalt eines Gemisches (G-f), umfassend mindestens ein Alkalioder Erdalkalisalz, sowie mindestens den Alkohol (A), das Lösungsmittel (L) und den mindestens einen ungesättigten Alkohol (B);
(g) Abtrennung des mindestens einen Alkali- oder Erdalkalisalzes aus dem in Stufe (f) erhaltenen Gemisch (G-f) unter Erhalt eines Gemisches (G-g), umfassend mindestens den Alkohol (A), das Lösungsmittel (L) und den mindestens einen ungesättigten Alkohol (B);
(h) Destillation des in Stufe (g) erhaltenen Gemisches (G-g) unter Erhalt des mindestens einen ungesättigten Alkohols (B), unter Erhalt einer Mischung (M-h), umfassend das Lösungsmittel (L) und den Alkohol (A), und unter Erhalt eines Gemisches (G-h), enthaltend geringe Mengen an dem mindestens einen ungesättigten Alkohol (B), wobei das Lösungsmittel (L) und der Alkohol (A) als Mischung in Stufe (a) rückgeführt werden und das Gemisch (G-h), enthaltend geringe Mengen an dem mindestens einen ungesättigten Alkohol (B), in Stufe (g) rückgeführt wird.

Darüber hinaus betrifft die vorliegende Erfindung auch ein integriertes Verfahren zur Herstellung mindestens eines hydrierten Alkohols (C), das die kontinuierlich durchgeführten Stufen (a) bis (g), wie oben beschrieben, aufweist sowie die kontinuierlich durchgeführten Stufen (g') und (h') umfaßt, die nach der Stufe (g) durchgeführt werden:
(g') Hydrierung des mindestens eines ungesättigten Alkohols (B) in dem aus Stufe (g) erhaltenen Gemisch (G-g) unter Erhalt eines Gemischs (G-g'), umfassend mindestens den Alkohol (A), das Lösungsmittel (L) und mindestens einen hydrierten Alkohol (C);
(h') Destillation des in Stufe (g') erhaltenen Gemisches (G-g') unter Erhalt des mindestens einen hydrierten Alkohols (C), unter Erhalt einer Mischung (M-h'), umfassend das Lösungsmittel (L) und den Alkohol (A), und unter Erhalt eines Gemisches (G-h'), enthaltend geringe Mengen an dem mindestens einen hydrierten Alkohol (C), wobei das Lösungsmittel (L) und der Alkohol (A) als Mischung in Stufe (a) rückgeführt werden und das Gemisch (G-h'), enthaltend geringe Mengen an dem mindestens einen hydrierten Alkohol (C), in Stufe (g) rückgeführt wird.

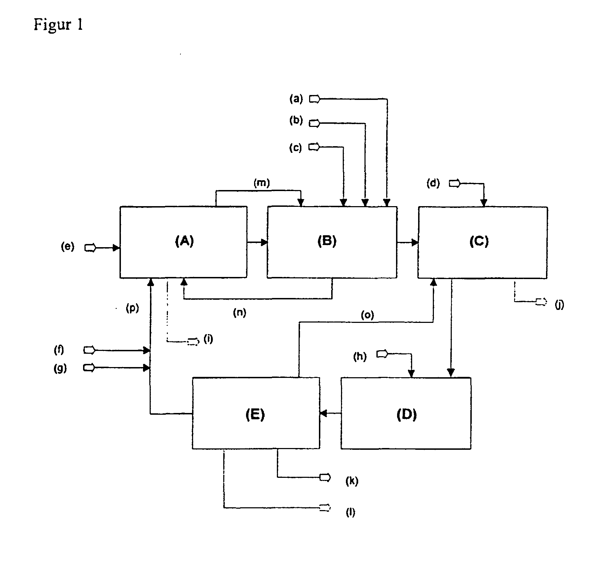

In Figur 1 ist eine Ausführungsform des erfindungsgemäßen Verfahrens zur Herstellung von DMHD dargestellt. Darin bezeichnen
(A) Basenpräparation
(B) Ethinylierung / Hydrolyse
(C) Salzabtrennung
(D) Hydrierung
(E) Reindestillation

(a) Aceton
(b) Acetylen
(c) Phosphorsäure
(d) Pluriol
(e) KOH
(f) Xylol
(g) i-Butanol
(h) Wasserstoff
(i) Leichtsieder
(j) Hochsieder
(k) DMHD → Konfektionierung
(l) Mittelsieder
(m) Wasser
(n) KOH
(o) Hochsieder
(p) Xylol/i-Butanol

Die folgenden Beispiele sollen der näheren Erläuterung des erfindungsgemäßen Verfahrens dienen.

### Beispiele

### Beispiel 1: Herstellung von Kaliumisobutylat

Der Versuch wurde auf einer Laborkolonne Innendurchmesser 80 mm mit 40 Glockenböden gefahren. Der Arbeitsdruck der Kolonne betrug 900 mbar.

Am Kopf der Kolonne wurden die anfallenden Brüden mittels eines Kondensators auskondensiert und in einen auf 50 °C temperierten Phasenscheider geleitet. Die organische Phase wurde aus dem Phasenscheider zurück in die Kolonne gefahren, die wäßrige Phase wurde standgeregelt in ein Vorlagegefäß abgezogen.

Der Sumpf der Kolonne bestand aus einem Doppelmantelrührbehälter, der mit Wärmeträgeröl von 218 °C Temperatur beheizt wurde. Der Sumpfaustrag der Kolonne erfolgte standgeregelt mittels Bodenablaßventil in eine Vorlage.

Auf den obersten Boden der Kolonne wurden 220 g/h einer wäßrigen Lösung von Kaliumhydroxid der Konzentration 33 Gew.-% zugefahren. Die Temperatur auf dem vierten Boden der Kolonne wurde gemessen und durch Zudosierung einer Lösung von 50 Gew.-% Isobutanol und 50 Gew.-% eines kommerziellen Xylolisomerengemischs auf 130 °C geregelt.

Die Kopftemperatur betrug bei dem Versuch 91 °C, die Sumpftemperatur 140°C. Nach Zulauf von insgesamt 4857 g einer 33 gew.-%igen KOH 33 und 4060 g Lösungsmittelgemisch aus 50 Gew.-% Iso-Butanol und 50 Gew.-% Xylol wurden 4727 g Kaliumisobutylatsuspension entnommen. Der KOH-Umsatz wurde zu 99,47% bestimmt, der KOiBu-Gehalt der Suspension wurde zu 5,52 mol/kg bestimmt. Am Phasenscheider wurden 3784 g wäßrige Phase entnommen.

### Beispiel 2: Herstellung von Kaliumisobutylat

Der Versuch wurde in analoger Weise zu Beispiel 1 durchgeführt.

Die Sumpftemperatur betrug 140 °C, der Ölmantel des Sumpfbehälters wurde bei 222 °C betrieben. Der Kolonnenkopfdruck betrug 900 mbar, die Kopftemperatur betrug 91 °C. Der Phasenscheider im Kopf wurde auf 50 °C thermostatisiert.

Auf den obersten Boden der Kolonne wurden 220 g/h wäßrige Kalilauge mit einer Konzentration von 33 Gew.-% KOH zugefahren. Auf den obersten Boden der Kolonne wurde ein Gemisch von 45 Gew.-% Isobutanol und 55 Gew.-% Orthoxylol gefahren. Der vierte Boden wurde durch Regelung der Zulaufmenge des Isobutanol-Xylol-Gemischs auf 130°C geregelt.

Insgesamt wurden 4328 g Isobutanol und 3194 g einer 33 gew.-%igen KOH zugefahren. Im Sumpf der Kolonne wurden 4707 g Kaliumisobutylatsuspension entnommen, im Phasenscheider der Kolonne wurden 2663 g wäßrige Phase ausgetragen. Die wäßrige Phase enthielt 5,37 Gew.-% Iso-Butanol.

Der Umsatz der KOH betrug 97,16 %, der Isobutylatgehalt der Suspension betrug 4,37 mol/kg.

### Beispiel 3: Herstellung von Kaliumisobutylat

Der Versuch wurde in analoger Weise zu Beispiel 1 und 2 durchgeführt.

Die Temperatur im Sumpf betrug 140 °C, die Ölbadtemperatur 222 °C. Als Kopftemperatur stellte sich bei einem Druck von 900 mbar 91 °C ein.

Als Zuläufe wurde rückgeführte Kalilauge aus der Hydrolyse des Ethinylierungsaustrags und Lösungsmittelgemisch aus der Reindestillation eingesetzt.

Die Konzentration der Kalilauge betrug 32,67 Gew.-%, die Lauge enthielt als Hauptverunreinigung 0,38 Gew.-% Dimethylhexindiol. Die Zusammensetzung des Lösungsmittelgemischs betrug 39,2 Gew.-% Isobutanol, 48,1 Gew.-% Xylol, 4,5 Gew.-% Wasser und 2,4 Gew.-% tert-Amylalkohol.

Die Zulaufmenge der Kalilauge betrug 220 g/h, die Zulaufinenge an Lösungsmittelgemisch wurde durch Regelung der Temperatur auf dem vierten Boden der Kolonne auf 130 °C eingestellt.

Nach Zugabe von 3585 g Kalilauge und 4026 g Lösungsmittelgemisch waren 4755 g Kaliumisobutylatsuspension und 3219 g Destillationswasser ausgetragen. Der KOH-Umsatz betrug 98,85 %, die Konzentration der Base betrug 4,69 mol/kg KOiBu. Das Destillationswasser enthielt als Hauptverunreinigung 2,8 Gew.-% Isobutanol, 2,0 % Methylbutanol und 0,9 % Aceton.

### Beispiel 4: Ethinylierung (Synthese von Dimethylhexindiol (DMHDY) durch kontinuierliche Ethinylierung von Aceton)

Als Reaktor diente ein 11-Schott-Doppelmantelglasreaktor, ausgerüstet mit einem doppelten Schrägblattrührer. Der Rührer wird mit 600 U/min betrieben. Die Kühlung der Reaktionsmischung erfolgte in einem externen Umpumpkreis, bestehend aus einer Zahnradpumpe und einem Doppelmantelrohr-Kühler. Die Pumpenleistung betrug zwischen 1 l/min und 4 l/min. Das gesamte, genutzte Reaktionsvolumen betrug 1,09 l.

In den Reaktor wurden nach einer Anfahrphase kontinuierlich 323 g/h Aceton, 73 g/h Acetylen und 1219 g/h Kalium-Isobutylatsuspension (5 mol/kg) gepumpt. Die Dosierung aller drei Ströme erfolgte getaucht auf das untere der beiden Rührblätter. Die Reaktionstemperatur wurde durch Kühlung im Bereich von 30 °C bis 35 °C gehalten.

Es wurde kontinuierlich ein Massenstrom von 1615 g/h ausgetragen und getaucht in einen 0,25 1-Schott-Doppelmantelglasreaktor mit einem zweistufigen Rührer eingetragen. Gleichzeitig und getrennt wurden 670 g/h Wasser in den Reaktor dosiert. Der Rührer des Reaktors wurde mit 800 U/min betrieben. Über einen freien Überlauf wurde die Mischung in einen Phasenscheider geleitet. Aus diesem werden 808 g/h wäßrige Phase und 1555 g/h organische Phase ausgetragen.

Die organische Phase enthielt 363 g/h Dimethylhexindiol (DMHDY) (Ausbeute 92 %) und 10 g/h Aceton (Umsatz 97 %) und einen Rest-KOH-Gehalt von ca. 90 g/h. Die wäßrige Phase enthielt 8 g/h an organischen Substanzen und 260 g/h KOH.

Die organische Phase wurde in einer zweistufigen Mixer-Settler-Anlage mit Wasser im Gegenstrom extrahiert. Die extrahierte organische Phase (1490 g/h) enthielt einen Restgehalt von 0,005 g/h KOH und 130 g/h Wasser. Der Restgehalt KOH wurde mit Phosphorsäure (40% in Wasser) neutralisiert.

### Beispiel 5: Herstellung von Dimethylhexindiol

In eine Reaktionsmischpumpe mit einem Nennvolumen von 20 ml wurden innerhalb einer Stunde kontinuierlich und synchron 529 g einer Kaliumisobutylat/Xylol-Suspension (4,5 Mol/kg), 127 g Aceton und 29 g Acetylengas eindosiert.das in der Reaktionsmischpumpe sehr gut durchgemischte Reaktionsgemisch wurde über einen Umpumpkreis geführt, in dem sich ein Wärmetauscher befand. Die Reaktionstemperatur in der Pumpe betrug 35 °C, vor dem Wärmetauscher 32 °C, nach dem Wärmetauscher 28 °C. Der Volumenstrom im Umpumpkreis lag im Bereich 80 bis 100 l/h. Aus dem Umpumpkreis heraus führte eine Nachreaktionstrecke in einen Hydrolysekessel, wo das Reaktionsgemisch mit 500 g/h Wasser hydrolysiert wurde. Nach Abtrennung der organischen Phase und Neutralisation mit 22,8 g Phosphorsäure erhielt man Dimethylhexindiol mit einem Umsatz von 98 % und einer Ausbeute von 90 %. Als Nebenprodukt wurde Methylbutinol mit einer Ausbeute von 2,3 % identifiziert.

### Beispiel 6: Herstellung von Methylbutinol

In einer Reaktionsmischpumpe mit einem Nennvolumen von 20 ml wurden innerhalb einer Stunde kontinuierlich und synchron 529 g einer Kaliumisobutylat/Xylol-Suspension (4,5 Mol/kg), 127 g Aceton und 58 g Acetylengas eindosiert. Das in der Reaktionsmischpumpe sehr gut durchgemischte Reaktionsgemisch wurde über einen Umpumpkreis geführt, in dem sich ein Wärmetauscher befand. Das Reaktionsgemisch wurde dadurch auf einer Reaktionstemperatur von 20 °C gehalten. Der Volumenstrom im Umpumpkreis lag im Bereich 80 bis 100 l/h. Aus dem Umpumpkreis heraus führte eine Nachreaktionstrecke in einen Hydrolysekessel, wo das Reaktionsgemisch mit 500 g/h Wasser hydrolysiert wurde. Nach Abtrennung der organischen Phase und Neutralisation mit 22,8 g Phosphorsäure erhielt man Methylbutinol mit einem Umsatz von 98 % und einer Ausbeute von 79 %. Als Nebenprodukt wird Dimethylhexindiol mit einer Ausbeute von 14 % identifiziert.

### Beispiel 7: Herstellung von Methylbutinol mit katalytischen Mengen Base

In einer Reaktionsmischpumpe mit einem Nennvolumen von 20 ml wurden innerhalb einer Stunde kontinuierlich und synchron 52,9 g einer Kaliumisobutylat/Xylol-Suspension (0,45 Mol/kg), 127 g Aceton (2,18 mol) und 114 g Acetylengas (4,4 mol) eindosiert. Das in der Reaktionsmischpumpe sehr gut durchgemischte Reaktionsgemisch wurde über einen Umpumpkreis geführt, in dem sich ein Wärmetauscher befand. Das Reaktionsgemisch wurde dadurch auf einer Reaktionstemperatur von 10 °C gehalten. Der Volumenstrom im Umpumpkreis lag im Bereich 80 bis 100 l/h. Aus dem Umpumpkreis heraus führte eine Nachreaktionstrecke in einen Hydrolysekessel, wo das Reaktionsgemisch mit 500 g/h Wasser hydrolysiert wurde. Nach Abtrennung der organischen Phase und Neutralisation mit 2,3 g Phosphorsäure erhielt man Methylbutinol mit einem Umsatz von 98 % und einer Ausbeute von 85 %. Als Nebenprodukt wird Dimethylhexindiol mit einer Ausbeute von 3 % identifiziert.

### Beispiel 8: Salzabtrennung durch Totaldestillation (Salzabtrennung aus der extrahierten Roh-DMHDY (Dimethylhexindiol)-Lösung durch Totalverdampfung)

Bei der Totalverdampfung der extrahierten Roh-DMHDY-Lösung aus Beispiel 4 wurde ein Dünnschicht- oder Fallfilmverdampfer mit Umlauf und einer zweistufigen Kondensation benutzt.

Die extrahierte Roh-DMHDY-Lösung wurde mit einer Flußrate von 743 g/h (172 g/h DMHDY) in einen bei 150 mbar und 170 °C betriebenen Verdampfer gepumpt. Dem Zulauf wurden 7 g/h eines hochsiedenden Sumpfverflüssigers (PLURIOL®) zugemischt. 722 g/h Brüden (168 g/h DMHDY) wurden in zwei, bei 40 °C und 5 °C temperierten Kondensatoren aufgefangen. In einer bei -78 °C betriebenen Kühlfalle wurden 4 g/h organische Substanz (Xylole, Isobutanol) abgeschieden. Der gut fließfähige Sumpf (5 g/h) wurd zu 60-80 % in den Prozeß rückgeführt.

Aus der organischen Phase wurden nach der Kondensation in einem Trenngefäß 12 g/h Wasser abgetrennt. Die Destillationsausbeute betrugt 97%.

### Beispiel 9: Hydrierung der totalverdampften Roh-DMHDY-Lösung zu Dimethylhexandiol (DMHD)

Die Hydrierung der Roh-DMHDY-Lösung wurde kontinuierlich an einem kugelförmigen Trägerkatalysator (Durchmesser: 2-4 mm, 0,25 % Pd auf Al₂O₃) in zwei hintereinander geschalteten Rohrreaktoren durchgeführt. Die Reaktoren hatten einen freien Reaktorquerschnitt von 656 mm², eine Länge von 1,7 m und ein befüllbares Volumen von 800 ml. Die Reaktoren waren begleitbeheizt. Die Innentemperatur der Reaktoren konnte am Kopf, in der Mitte und im unteren Teil der Reaktoren gemessen werden. Beide Reaktoren waren mittig mit 250 ml Katalysator befüllt, das restliche Volumen war mit inerten Glaskugel gefüllt.

Nach dem Anfahren der Reaktoren wurde 110 g/h Zulauf (25,5 g/h DMHDY) bei 80 °C auf den ersten Reaktor dosiert. Wasserstoff wurde ebenfalls von oben nach unten druckgeregelt bei 30 bar zugefahren. Ein konstanter Abgasstrom von 2 l/h wurde eingestellt.

Nach Verlassen des Reaktors wurde ein Teil des Austrages wieder auf den Kopf des ersten Reaktors gepumpt. Die Umpumpmenge betrug 50 l/h. Der andere Teil wurd von unten nach oben in den zweiten Reaktor gepumpt. Wasserstoff gelangte druckgeregelt bei 30 bar ebenfalls von unten nach oben über den zweiten Reaktor. Der Abgasstrom betrugt 1 l/h. Der zweite Reaktor besaß keinen Flüssigkeitsumlauf. Nach Verlassen des zweiten Reaktors wurde der Austrag (116,2 g/h) in einen Behälter entspannt.

Die Ausbeute an DMHD beträgt 98,5 %.

### Beispiel 10: Reindestillation

Der Versuch wurde auf einer Laborkolonne mit Innendurchmesser 50 mm gefahren. Der mittlere Teil der Kolonne war mit einer Trennwand in zwei symmetrische Teile getrennt.

Der untere, ungeteilte Abschnitt der Kolonne (30 cm Höhe) war mit Gewebepackung (1200 m²/m³ Oberfläche) gefüllt. Der geteilte Bereich der Kolonne (90 cm Höhe) war mit Drahtringen (Durchmesser 3 mm) gefüllt. Über dem mittleren Teil der Kolonne war ein Schwenktrichter angebracht, der den Flüssigkeitsstrom (Aufteilungsverhältnis Zulaufseite : Abzugsseite von 3:7) aufteilte. Der oberste Teil der Kolonne (Höhe 60 cm) war mit Laborgewebepackung (1200 m²/m³) bestückt.

Der Kopfdruck der Kolonne betrug 200 mbar. Die Sumpftemperatur wurde durch die Sumpfabzugsmenge auf 185 °C geregelt, die Temperatur der Sumpfheizung betrug 205 °C. Die Seitenabzugsmenge wurde durch Regelung des Sumpfstandes eingestellt. Das Rücklaufverhältnis wurde durch Regelung der Temperatur in der Mitte des oberen Kolonnenabschnittes eingestellt.

Als Zulaufmenge wurden 150 g/h eingestellt. Die Regelungstemperatur im oberen Teil der Kolonne betrug 120 °C. Insgesamt wurden 3567 g eines Gemisches der Zusammensetzung 60 Gew.-% Isobutanol, 13 Gew.-% Xylol, 13,1 Gew.-% Dimethylhexandiol und 8 Gew.-% Wasser zugefahren. Darüber hinaus waren im Zulaufgemisch verschiedene Spurenkomponenten, hauptsächlich Kondensationsprodukte des Acetons enthalten.

Über Kopf wurden 3117 g eines Leichtsiedergemisches mit einem Restgehalt von 0,04 % Dimethylhexandiol genommen. Im Seitenabzug wurden 476 g Dimethylhexandiol der Reinheit 99,72 Gew.-% gewonnen. Die Farbzahl des Produkts betrug 20 APHA.

Während des Bilanzzeitraumes wurden keine Hochsieder aus dem Sumpf gefahren.

### Beispiel 11: Reindestillation

Der Versuch wurde analog dem Beispiel 1 durchgeführt.

Als Zulaufmenge wurden 150 g/h eingestellt. Die Sumpftemperatur betrug 202 °C, die Sumpftemperatur wurde bei 185 °C geregelt.

Das Zulaufgemisch hatte die Zusammensetzung: 30 Gew.-% Isobutanol, 23,8 Gew.-% Dimethylhexandiol, 34 Gew.-% Xylole, 2 Gew.-% Methylbutanol, 8 Gew.-% Wasser. Darüberhinaus waren im Zulaufgemisch verschiedene Spurenkomponenten, hauptsächlich Kondensationsprodukte des Acetons enthalten.

Über Kopf wurden 2636 g Leichtsieder mit einem Gehalt an Dimethylhexandiol von 2,2 % gefahren. Im Seitenabzug wurden 764 g Dimethylhexandiol mit einer Reinheit von 99,90 % und einer Farbzahl von 9 APHA gewonnen. Im Sumpf wurden während des Versuchszeitraumes 11 g Hochsieder mit einem Gehalt an DMHD von 17,1 Gew.-% abgezogen.

## Patentansprüche

1. Verfahren zur Herstellung mindestens eines ungesättigten Alkohols (B), das die folgenden Stufen (I) bis (III) umfaßt:
(I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
(II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
(III) Destillation des in Stufe (II) erhaltenen Gemisches (G-II) unter Erhalt des mindestens einen Alkohols (B) sowie eines Gemisches (G-III), umfassend das Lösungsmittel (L) und den Alkohol (A),
wobei das in Stufe (III) erhaltene Lösungmittel (L) und der in Stufe (III) erhaltene Alkohol (A) als Mischung in Stufe (I) rückgeführt werden, **dadurch gekennzeichnet, daß** die Stufen (I) bis (III) kontinuierlich durchgeführt werden.

2. Verfahren zur Herstellung mindestens eines hydrierten Alkohols (C), das die folgenden Stufen (I) bis (III') umfaßt :
(I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
(II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
(II') Hydrierung mindestens eines ungesättigten Alkohols (B) in dem aus Stufe (II) erhaltenen Gemisch (G-II) unter Erhalt eines Gemisches (G-II'), umfassend mindestens einen hydrierten Alkohol (C), den Alkohol (A) und das Lösungsmittel (L);
(III') Destillation des in Stufe (II') erhaltenen Gemisches (G-II') unter Erhalt des mindestens einen Alkohols (C) sowie eines Gemisches (G-III'), umfassend das Lösungsmittel (L) und den Alkohol (A),
**dadurch gekennzeichnet, daß** das in Stufe (III') erhaltene Lösungsmittel (L) und der in Stufe (III') erhaltene Alkohol (A) als Mischung in Stufe (I) rückgeführt werden.

3. Verfahren nach Anspruch 1 oder 2, **dadurch gekennzeichnet, daß** die Stufe (II) die folgenden Schritte (i) bis (v) umfaßt:
(i) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt einer Mischung (M-i);
(ii) Hydrolyse der aus Schritt (i) erhaltenen Mischung (M-i) unter Erhalt einer mehrphasigen Mischung (M-ii), umfassend mindestens eine organische Phase;
(iii) Abtrennung der mindestens einen organischen Phase aus der in Schritt (ii) erhaltenen mehrphasigen Mischung (M-ii);
(iv) Extraktion der mindestens einen in Schritt (iii) abgetrennten organischen Phase;
(v) Neutralisation der mindestens einen, in Schritt (iv) extrahierten organischen Phase unter Erhalt einer Mischung (M-v), umfassend mindestens ein Alkali- oder Erdalkalisalz;
(vi) Abtrennung des mindestens einen Alkali- oder Erdalkalisalzes aus der in Schritt (v) erhaltenen Mischung (M-v) unter Erhalt eines Gemisches (G-II), umfassend mindestens den Alkohol (A), das Lösungsmittel (L) und einen ungesättigten Alkohol (B).

4. Verfahren nach Anspruch 3, **dadurch gekennzeichnet, daß** die Extraktion in Schritt (iv) als Gegenstromextraktion durchgeführt wird.

5. Verfahren nach einem der Ansprüche 1 bis 4, das die weitere Stufe (IV) umfaßt:
(IV) Konfektionierung des mindestens einen Alkohols B oder C, der in der Stufe (II) oder in den Stufen (II) und (II') hergestellt und in Stufe (III) oder in Stufe (III') erhalten wird.

6. Verfahren nach einem der Ansprüche 1 bis 5, **dadurch gekennzeichnet, daß** die Reste R und R' der Carbonylverbindung der allgemeinen Struktur R-CO-R' gleich oder unterschiedlich sind und geradkettige, verzweigtkettigte oder cyclische, gegebenfalls ungesättigte aliphatische Reste sind.

7. Verfahren nach Anspruch 6, **dadurch gekennzeichnet, daß** die Carbonylverbindung der allgemeinen Struktur R-CO-R' Aceton oder Methyl-iso-Butylketon ist.

8. Verfahren nach einem der Ansprüche 1 bis 7, **dadurch gekennzeichnet, daß** als Alkin der allgemeinen Struktur R"-C≡C-H Acetylen eingesetzt wird.

9. Integriertes Verfahren zur Herstellung mindestens eines ungesättigten Alkohols (B), das die folgenden kontinuierlich durchgeführten Stufen (a) bis (h) umfaßt:
(a) Umsetzung mindestens eines Alkali- oder Erdalkalihydroxids mit mindestens einem Alkohol (A) in mindestens einem organischen Lösungsmittel (L) unter Verwendung der in Stufe (d) erhaltenen wäßrigen Phase (P-d) und unter Erhalt eines Gemisches (G-a), umfassend mindestens das Lösungsmittel (L), den Alkohol (A) und ein Alkoholat (AL), und unter Erhalt einer wäßrigen Phase (P-a), die der Stufe (e) zugeführt wird;
(b) Umsetzung mindestens einer Carbonylverbindung der allgemeinen Struktur R-CO-R' mit mindestens einem Alkin der allgemeinen Struktur R"-C≡C-H und dem in Stufe (a) erhaltenen Gemisch (G-a) unter Erhalt eines Gemisches (G-b), umfassend mindestens einen ungesättigten Alkohol (B), wobei die Umsetzung vorzugsweise in einer Reaktionsmischpumpe durchgeführt wird;
(c) Hydrolyse des Gemisches (G-b) aus Stufe (b) unter Verwendung der in Stufe (e) erhaltenen wäßrigen Phase (P-e) unter Erhalt einer mehrphasigen Mischung (M-c), umfassend mindestens eine organische Phase und mindestens eine wäßrige Phase;
(d) Abtrennung der mindestens einen organischen Phase aus der in Stufe (c) erhaltenen mehrphasigen Mischung (M-c) unter Erhalt mindestens einer wäßrigen Phase (P-d), die in Stufe (a) rückgeführt wird;
(e) Gegenstromextraktion der in Stufe (d) abgetrennten mindestens einen organischen Phase unter Verwendung der in Stufe (a) erhaltenen wäßrigen Phase (P-a) und unter Erhalt einer wäßrigen Phase (P-e), die in Stufe (c) rückgeführt wird;
(f) Neutralisation der mindestens einen, in Stufe (e) erhaltenen organischen Phase unter Erhalt eines Gemisches (G-f), umfassend mindestens ein Alkali- oder Erdalkalisalz, sowie mindestens den Alkohol (A), das Lösungsmittel (L) und den mindestens einen ungesättigten Alkohol (B);
(g) Abtrennung des mindestens einen Alkali- oder Erdalkalisalzes aus dem in Stufe (f) erhaltenen Gemisch (G-f) unter Erhalt eines Gemisches (G-g), umfassend mindestens den Alkohol (A), das Lösungsmittel (L) und den mindestens einen ungesättigten Alkohol (B);
(h) Destillation des in Stufe (g) erhaltenen Gemisches (G-g) unter Erhalt des mindestens einen ungesättigten Alkohols (B), unter Erhalt einer Mischung (M-h), umfassend das Lösungsmittel (L) und den Alkohol (A), und unter Erhalt eines Gemisches (G-h), enthaltend geringe Mengen an dem mindestens einen ungesättigten Alkohol (B), wobei das Lösungsmittel (L) und der Alkohol (A) als Mischung in Stufe (a) rückgeführt werden und das Gemisch (G-h), enthaltend geringe Mengen an dem mindestens einen ungesättigten Alkohol (B), in Stufe (g) rückgeführt wird.

10. Integriertes Verfahren zur Herstellung mindestens eines hydrierten Alkohols (C), das die kontinuierlich durchgeführten Stufen (a) bis (g) gemäß Anspruch 9 aufweist sowie die kontinuierlich durchgeführten Stufen (g') und (h') umfaßt, die nach der Stufe (g) durchgeführt werden:
(g') Hydrierung des mindestens eines ungesättigten Alkohols (B) in dem aus Stufe (g) erhaltenen Gemisch (G-g) unter Erhalt eines Gemischs (G-g'), umfassend mindestens den Alkohol (A), das Lösungsmittel (L) und mindestens einen hydrierten Alkohol (C);
(h') Destillation des in Stufe (g') erhaltenen Gemisches (G-g') unter Erhalt des mindestens einen hydrierten Alkohols (C), unter Erhalt einer Mischung (M-h'), umfassend das Lösungsmittel (L) und den Alkohol (A), und unter Erhalt eines Gemisches (G-h'), enthaltend geringe Mengen an dem mindestens einen hydrierten Alkohol (C), wobei das Lösungsmittel (L) und der Alkohol (A) als Mischung in Stufe (a) rückgeführt werden und das Gemisch (G-h'), enthaltend geringe Mengen an dem mindestens einen hydrierten Alkohol (C), in Stufe (g) rückgeführt wird.

## Claims

1. A process for preparing at least one unsaturated alcohol (B), which comprises the steps (I) to (III) below:
(I) reaction of at least one alkali metal hydroxide or alkaline earth metal hydroxide with at least one alcohol (A) in at least one organic solvent (L) to give a mixture (G-I) comprising at least the alcohol (A), the solvent (L) and an alkoxide (AL);
(II) reaction of at least one carbonyl compound of the formula R-CO-R' with at least one alkyne of the formula R"-C≡C-H and the mixture (G-I) obtained in step (I) to give a mixture (G-II) comprising at least the alcohol (A), the solvent (L) and an unsaturated alcohol (B);
(III) distillation of the mixture (G-II) obtained in step (II) to give the alcohol or alcohols (B) and a mixture (G-III) comprising the solvent (L) and the alcohol (A),
where the solvent (L) obtained in step (III) and the alcohol (A) obtained in step (III) are recycled as a mixture to step (I), wherein steps (I) to (III) are carried out continuously.

2. A process for preparing at least one hydrogenated alcohol (C), which comprises the steps (I) to (III') below:
(I) reaction of at least one alkali metal hydroxide or alkaline earth metal hydroxide with at least one alcohol (A) in at least one organic solvent (L) to give a mixture (G-I) comprising at least the alcohol (A), the solvent (L) and an alkoxide (AL);
(II) reaction of at least one carbonyl compound of the formula R-CO-R' with at least one alkyne of the formula R"-C≡C-H and the mixture (G-I) obtained in step (I) to give a mixture (G-II) comprising at least the alcohol (A), the solvent (L) and an unsaturated alcohol (B);
(II') hydrogenation of at least one unsaturated alcohol (B) in the mixture obtained from step (II) to give a mixture (G-II') comprising at least one hydrogenated alcohol (C), the alcohol (A) and the solvent (L);
(III') distillation of the mixture (G-II') obtained in step (II') to give the alcohol or alcohols (C) and a mixture (G-III') comprising the solvent (L) and the alcohol (A),
wherein the solvent (L) obtained in step (III') and the alcohol (A) obtained in step (III') are recycled as a mixture to step (I).

3. A process as claimed in claim 1 or 2, wherein the step (II) comprises the substeps (i) to (vi) below:
(i) reaction of at least one carbonyl compound of the formula R-CO-R' with at least one alkyne of the formula R"-C≡C-H and the mixture (G-I) obtained in step (I) to give a mixture (M-i);
(ii) hydrolysis of the mixture (M-i) obtained from substep (i) to give a multiphase mixture (M-ii) comprising at least one organic phase;
(iii) separation of the organic phase or phases from the multiphase mixture (M-ii) obtained in substep (ii);
(iv) extraction of the organic phase or phases separated off in step (iii);
(v) neutralization of the organic phase or phases extracted in step (iv) to give a mixture (M-v) comprising at least one alkali metal salt or alkaline earth metal salt;
(vi) separation of the alkali metal salt(s) or alkaline earth metal salt(s) from the mixture (M-v) obtained in substep (v) to give a mixture (G-II) comprising at least the alcohol (A), the solvent (L) and an unsaturated alcohol (B).

4. A process as claimed in claim 3, wherein the extraction in substep (iv) is carried out as a countercurrent extraction.

5. A process as claimed in any of claims 1 to 4 which comprises the further step (IV):
(IV) finishing of the alcohol or alcohols B or C prepared in step (II) or in steps (II) and (II') and obtained in step (III) or in step (III').

6. A process as claimed in any of claims 1 to 5, wherein the radicals R and R' of the carbonyl compound of the formula R-CO-R' are identical or different and are straight-chain, branched or cyclic, saturated or unsaturated aliphatic radicals.

7. A process as claimed in claim 6, wherein the carbonyl compound of the formula R-CO-R' is acetone or methyl isobutyl ketone.

8. A process as claimed in any of claims 1 to 7, wherein the alkyne of the formula R"-C≡C-H is acetylene.

9. An integrated process for preparing at least one unsaturated alcohol (B), which comprises the continuously operated steps (a) to (h) below:
(a) reaction of at least one alkali metal hydroxide or alkaline earth metal hydroxide with at least one alcohol (A) in at least one organic solvent (L) using the aqueous phase (P-d) obtained in step (d) to give a mixture (G-a) comprising at least the solvent (L), the alcohol (A) and an alkoxide (AL) and to give an aqueous phase (P-a) which is passed to step (e);
(b) reaction of at least one carbonyl compound of the formula R-CO-R' with at least one alkyne of the formula R"-C≡C-H and the mixture (G-a) obtained in step (a) to give a mixture (G-b) comprising at least one unsaturated alcohol (B), where the reaction is preferably carried out in a reaction mixing pump;
(c) hydrolysis of the mixture (G-b) from step (b) using the aqueous phase (P-e) obtained in step (e) to give a multiphase mixture (M-c) comprising at least one organic phase and at least one aqueous phase;
(d) separation of the organic phase or phases from the multiphase mixture (M-c) obtained in step (c) to give at least one aqueous phase (P-d) which is recycled to step (a);
(e) countercurrent extraction of the organic phase or phases separated off in step (d) using the aqueous phase (P-a) obtained in step (a) to give an aqueous phase (P-e) which is recycled to step (c);
(f) neutralization of the organic phase or phases obtained in step (e) to give a mixture (G-f) comprising at least one alkali metal salt or alkaline earth metal salt and also at least the alcohol (A), the solvent (L) and the unsaturated alcohol or alcohols (B);
(g) separation of the alkali metal salt(s) or alkaline earth metal salt(s) from the mixture (G-f) obtained in step (f) to give a mixture (G-g) comprising at least the alcohol (A), the solvent (L) and the unsaturated alcohol or alcohols (B);
(h) distillation of the mixture (G-g) obtained in step (g) to give the unsaturated alcohol or alcohols (B), to give a mixture (M-h) comprising the solvent (L) and the alcohol (A) and to give a mixture (G-h) comprising small amounts of the unsaturated alcohol or alcohols (B), where the solvent (L) and the alcohol (A) are recycled as a mixture to step (a) and the mixture (G-h) comprising small amounts of the unsaturated alcohol or alcohols (B) is recycled to step (g).

10. An integrated process for preparing at least one hydrogenated alcohol (C), which comprises the continuously operated steps (a) to (g) as claimed in claim 9 and also the continuously operated steps (g') and (h') which are carried out after step (g):
(g') hydrogenation of the unsaturated alcohol or alcohols (B) in the mixture (G-g) obtained from step (g) to give a mixture (G-g') comprising at least the alcohol (A), the solvent (L) and at least one hydrogenated alcohol (C);
(h') distillation of the mixture (G-g') obtained in step (g') to give the hydrogenated alcohol or alcohols (C), to give a mixture (M-h') comprising the solvent (L) and the alcohol (A) and to give a mixture (G-h') comprising small amounts of the hydrogenated alcohol or alcohols (C), where the solvent (L) and the alcohol (A) are recycled as a mixture to step (a) and the mixture (G-h') comprising small amounts of the hydrogenated alcohol or alcohols (C) is recycled to step (g).

## Revendications

1. Procédé de préparation d'au moins un alcool insaturé (B), comprenant les étapes (I) à (III) suivantes :
(I) Réaction d'au moins un hydroxyde de métal alcalin ou alcalino-terreux avec au moins un alcool (A) dans au moins un solvant organique (L), avec obtention d'un mélange (G-I) comprenant au moins l'alcool (A), le solvant (L) et un alcoolate (AL) ;
(II) Réaction d'au moins un composé carbonyle de la structure générale R-CO-R' avec au moins un alcyne de la structure générale R"-C≡C-H et le mélange (G-I) obtenu à l'étape (I), avec obtention d'un mélange (G-II) comprenant au moins l'alcool (A), le solvant (L) et un alcool insaturé (B) ;
(III) Distillation du mélange (G-II) obtenu à l'étape (II), avec obtention de l'au moins un alcool (B) ainsi que d'un mélange (G-III) comprenant le solvant (L) et l'alcool (A),
dans lequel le solvant (L) obtenu à l'étape (III) et l'alcool obtenu (A) à l'étape (III) sont recyclés sous forme mélangée vers l'étape (I), **caractérisé en ce que** les étapes (I) à (III) s'effectuent en continu.

2. Procédé de préparation d'au moins un alcool hydrogéné (C), comprenant les étapes (I) à (III') suivantes :
(I) Réaction d'au moins un hydroxyde de métal alcalin ou alcalino-terreux avec au moins un alcool (A) dans au moins un solvant organique (L), avec obtention d'un mélange (G-I) comprenant au moins l'alcool (A), le solvant (L) et un alcoolate (AL) ;
(II) Réaction d'au moins un composé carbonyle de la structure générale R-CO-R' avec au moins un alcyne de la structure générale R"-C≡C-H et le mélange (G-I) obtenu à l'étape (I), avec obtention d'un mélange (G-II) comprenant au moins l'alcool (A), le solvant (L) et un alcool insaturé (B) ;
(II') Hydrogénation d'au moins un alcool insaturé (B) dans le mélange (G-II) obtenu à l'étape (II), avec obtention d'un mélange (G-II') comprenant au moins un alcool hydrogéné (C), l'alcool (A) et le solvant (L) ;
(III') Distillation du mélange (G-II') obtenu à l'étape (II'), avec obtention de l'au moins un alcool (C) ainsi que d'un mélange (G-III') comprenant le solvant (L) et l'alcool (A),
**caractérisé en ce que** le solvant (L) obtenu à l'étape (III') et l'alcool (A) obtenu à l'étape (III') sont recyclés sous forme mélangée vers l'étape (I).

3. Procédé selon la revendication 1 ou la revendication 2, **caractérisé en ce que** l'étape (II) comprend les étapes (i) à (v) suivantes :
(i) Réaction d'au moins un composé carbonyle de la structure générale R-CO-R' avec au moins un alcyne de la structure générale R"-C≡C-H et le mélange (G-I) obtenu à l'étape (I) avec obtention d'un mélange (M-i) ;
(ii) Hydrolyse du mélange (M-i) obtenu dans l'étape (i) avec obtention d'un mélange multiphasique (M-ii) comprenant au moins une phase organique ;
(iii) Séparation de l'au moins une phase organique d'avec le mélange multiphasique (M-ii) obtenu à l'étape (ii) ;
(iv) Extraction de l'au moins une phase organique séparée à l'étape (iii) ;
(v) Neutralisation de l'au moins une phase organique extraite à l'étape (iv) avec obtention d'un mélange (M-v) comprenant au moins un sel de métal alcalin ou alcalino-terreux ;
(vi) Séparation de l'au moins un sel de métal alcalin ou alcalino-terreux d'avec le mélange (M-v) obtenu à l'étape (v) avec obtention d'un mélange (G-II) comprenant au moins l'alcool (A), le solvant (L) et un alcool insaturé (B).

4. Procédé selon la revendication 3, **caractérisé en ce que** l'extraction à l'étape (iv) se fait sous la forme d'une extraction à contre-courant.

5. Procédé selon l'une des revendications 1 à 4, comprenant l'étape (IV) supplémentaire :
(IV) Confection de l'au moins un alcool B ou C qui est préparé à l'étape (II) ou dans les étapes (II) et (II') et à l'étape (III) ou bien obtenu à l'étape (III').

6. Procédé selon l'une des revendications 1 à 5, **caractérisé en ce que** les résidus R et R' du composé carbonyle de la structure générale R-CO-R' sont identiques ou différentes et représentent des résidus aliphatiques à chaîne droite, ramifiée ou cyclique, le cas échéant insaturés.

7. Procédé selon la revendication 6, **caractérisé en ce que** le composé carbonyle de la structure générale R-CO-R' est l'acétone ou la méthylisobutylcétone.

8. Procédé selon l'une des revendications 1 à 7, **caractérisé en ce que**, comme alcyne de la structure générale R"-C≡C-H, on utilise l'acétylène.

9. Procédé intégré de préparation d'au moins un alcool insaturé (B), comprenant les étapes (a) à (h) suivantes, effectuées en continu :
(a) Réaction d'au moins un hydroxyde de métal alcalin ou alcalino-terreux avec au moins un alcool (A) dans au moins un solvant organique (L) en utilisant la phase aqueuse (P-d) obtenue à l'étape (d), avec obtention d'un mélange (G-a) comprenant au moins le solvant (L), l'alcool (A) et un alcoolate (AL) et avec obtention une phase aqueuse (P-a) qui est amenée à l'étape (e) ;
(b) Réaction d'au moins un composé carbonyle de la structure générale R-CO-R' avec au moins un alcyne de la structure générale R"-C≡C-H et le mélange (G-a) à l'étape (a) obtenu avec obtention d'un mélange (G-b) comprenant au moins un alcool insaturé (B), la réaction se faisant de préférence dans une pompe mélangeuse de réaction ;
(c) Hydrolyse du mélange (G-b) obtenu à l'étape (b) en utilisant la phase aqueuse (P-e) obtenue à l'étape (e), avec obtention d'un mélange multiphasique (M-c) comprenant au moins une phase organique et au moins une phase aqueuse ;
(d) Séparation de l'au moins une phase organique d'avec le mélange multiphasique (M-c) obtenu à l'étape (c), avec obtention d'au moins une phase aqueuse (P-d) qui est recyclée vers l'étape (a) ;
(e) extraction à contre-courant de l'au moins une phase organique séparée à l'étape (d) en utilisant la phase aqueuse (P-a) obtenue à l'étape (a), avec obtention d'une phase aqueuse (P-e) qui est recyclée vers l'étape (c) ;
(f) Neutralisation de l'au moins une phase organique obtenue à l'étape (e), avec obtention d'un mélange (G-f) comprenant au moins un sel de métal alcalin ou alcalino-terreux ainsi qu'au moins l'alcool (A), le solvant (L) et l'au moins un alcool insaturé (B) ;
(g) Séparation de l'au moins un sel de métal alcalin ou alcalino-terreux d'avec le mélange (G-f) obtenu à l'étape (f), avec obtention d'un mélange (G-g) comprenant au moins l'alcool (A), le solvant (L) et l'au moins un alcool insaturé (B) ;
(h) Distillation du mélange (G-g) obtenu à l'étape (g) avec obtention de l'au moins un alcool insaturé (B), avec obtention d'un mélange (M-h) comprenant le solvant (L) et l'alcool (A), et avec obtention d'un mélange (G-h) contenant des quantités minimes de l'au moins un alcool insaturé (B), le solvant (L) et l'alcool (A) étant recyclés sous forme mélangée vers l'étape (a) et le mélange (G-h), contenant des quantités minimes de l'au moins un alcool insaturé (B), étant recyclé vers l'étape (g).

10. Procédé intégré de préparation d'au moins un alcool hydrogéné (C), comprenant les étapes (a) à (g) effectuées en continu selon la revendication 9 ainsi que les étapes (g') et (h') effectuées en continu, qui se font après l'étape (g) :
(g') Hydrogénation de l'au moins un alcool insaturé (B) dans le mélange (G-g) obtenu à l'étape (g), avec obtention d'un mélange (G-g') comprenant au moins l'alcool (A), le solvant (L) et au moins un alcool hydrogéné (C) ;
(f) Distillation du mélange (G-g') obtenu à l'étape (g'), avec obtention de l'au moins un alcool hydrogéné (C), avec obtention d'un mélange (M-h') comprenant le solvant (L) et l'alcool (A), et avec obtention d'un mélange (G-h') contenant des quantités minimes de l'au moins un alcool hydrogéné (C), le solvant (L) et l'alcool (A) étant recyclés sous forme mélangée vers l'étape (a) et le mélange (G-h'), contenant des quantités minimes de l'au moins un alcool hydrogéné (C), étant recyclé vers l'étape (g).
